# EUROPEAN PATENT SPECIFICATION

(11) **EP 4 489 709 B1**
(45) Date of publication and mention of the grant of the patent: **18.02.2026**
(21) Application number: 22710617.6
(22) Date of filing: 09.03.2022
(51) Int. Cl.: A61F 13/49, A61F 13/505

(54) **WASHABLE AND REUSABLE ABSORBENT UNDERGARMENT**
WASCHBARE UND WIEDERVERWENDBARE ABSORBIERENDE UNTERWÄSCHE
SOUS-VÊTEMENT ABSORBANT LAVABLE ET RÉUTILISABLE

(43) Date of publication of application: 15.01.2025
(73) Proprietor: Essity Hygiene and Health Aktiebolag, 405 03 Göteborg (SE)
(72) Inventor: FLODIN VELTMAN, Alice, 405 03 GÖTEBORG (SE); BLOMSTRÖM, Philip, 405 03 GÖTEBORG (SE); KNÖS, Anna, 405 03 GÖTEBORG (SE)
(74) Representative: Hoffmann Eitle
(86) International application number: PCT/EP2022/055972
(87) International publication number: WO 2023/169665

(56) References cited:
- JP-A- 2017 513 671
- US-A1- 2008 222 781
- US-A1- 2015 290 049

## Description

### TECHNICAL FIELD

The disclosure relates to a washable and reusable absorbent undergarment.

### BACKGROUND

An absorbent article, such as an absorbent undergarment, is worn for the purpose of absorbing body fluids such as urine and vaginal fluids. A washable and reusable absorbent undergarment comprises fabrics of woven or knitted materials. Conventionally such a washable and reusable absorbent undergarment comprises an integral absorbent assembly located in the crotch region of the wearer when the undergarment is worn. After use the absorbent undergarment is washed or laundered before reuse. The reusable absorbent undergarments have been mainly supplied for low to moderate volumes of body fluids. For sustainability reasons there has been an increased demand from users for reusable absorbent undergarments and consequently there is also a demand for such undergarments suitable for moderate to heavy flows of body fluids.

Also for reusable absorbent undergarments suitable for moderate to heavy flows of body fluids, there is a demand for the undergarment being comfortable to wear as well as providing a good fit and being aesthetically pleasing. As such, there is a desire to provide reusable absorbent undergarments suitable for moderate to heavy flows of body fluids, wherein the required capacity for absorbing body fluids is provided without unnecessary bulk and/or aesthetically disadvantageous effects such as unwanted folds and/or creases. Documents US 2015/290049, US 2008/222781 and JP 2017/513671 represent relevant prior art.

### SUMMARY

The object of the invention is to meet one or more of the above-mentioned needs, or to provide a useful alternative.

This object is met by a washable and reusable absorbent undergarment according to claim 1.

As such, there is provided a washable and reusable undergarment, comprising one or more fabric panels forming a front region, a back region and an intermediate region extending between the front and back regions. The front region and the back region are joined such that the undergarment forms a waist opening and a pair of leg openings. A central longitudinal axis of the undergarment is defined along the one or more fabric panels of the undergarment from the back region and towards the front region. A transversal crotch axis of the undergarment is defined in a direction extending between the leg openings and so as to divide the intermediate region into a front intermediate region extending longitudinally between the transversal crotch axis and a front end of each leg opening, and a rear intermediate region extending longitudinally between the transversal crotch axis and a rear end of each leg opening, the transversal crotch axis being perpendicular to the central longitudinal axis.

As such, the transversal crotch axis divides each leg opening into a leg opening front portion, which is intended to extend from the crotch of the user towards the front of the undergarment when the article is worn, and a leg opening rear portion, which is intended to extend from the crotch of the user towards the rear of the undergarment when the article is worn. The intermediate region extends longitudinally over the distance between a front end of the leg openings and a rear end of the leg openings. The front intermediate region extends longitudinally over the distance between the front end of the leg openings and the transversal crotch axis, and the rear intermediate region extends longitudinally over the distance between the rear end of the leg openings and the transversal crotch axis.

In view of the above, the central longitudinal axis will be located centrally as seen over a total transversal width of the undergarment. However, the transversal crotch axis is to be located centrally over the crotch portion of the undergarment, i.e. the portion of the undergarment intended to cover the crotch area of a user when worn. As such, the transversal crotch axis need not be located centrally as seen over a total longitudinal length of the undergarment.

The undergarment further comprises an absorbent assembly comprising a wearer facing top layer, a moisture barrier, and at least two intermediate layers being superimposed along a height axis, perpendicular to the central longitudinal axis and the transversal crotch axis, between the top layer and the moisture barrier. The absorbent assembly is permanently attached to at least one out of the one or more of fabric panels in at least part of the intermediate region by a set of one or more bonding members. Further, in the front intermediate region of the undergarment, a maximum front bond width over the set of bonding members attaching the absorbent assembly to the one or more fabric panels is no more than 11 cm.

As set out in the above, the washable and reusable undergarment proposed herein comprises an absorbent assembly comprising a wearer facing top layer, a moisture barrier, and at least two intermediate layers, i.e. the absorbent assembly comprises at least three layers plus the moisture barrier, which in some variants may be a fourth layer, i.e. a moisture barrier layer. Such an absorbent assembly is particularly suitable to provide an undergarment for absorbing relatively high flows, such as moderate to heavy flows of body fluids. However, on account of the relatively high number of layers in the absorbent assembly, the presence of the absorbent assembly may have an adverse effect on the capacity of the undergarment to provide a good fit conforming to the body of the user in the same manner as a regular undergarment.

As proposed herein, the maximum front bond width being defined as the maximum transversal width measured over the set of bonding members and in the front intermediate region of the undergarment, is no more than 11 cm. With this restriction to the maximum front bond width, it has been found that unwanted folds or creases as seen from the front side of the undergarment when worn by a user may be reduced or avoided.

As such, a washable and reusable undergarment is provided which is suitable for moderate to heavy flows of body fluids whilst at the same time providing a satisfactory fit to the body of the user with an outer appearance similar to a regular undergarment.

Optionally, the maximum front bond width may be no more than 10 cm, such as for example no more than 9 cm. With a lesser maximum front bond width, the tendency for creases forming on the front side of the undergarment when worn may be further reduced.

Optionally, the maximum front bond width in the front intermediate region may be no less than 4 cm, such as no less than 5 cm or no less than 6 cm. As such, a suitable balance between the desire to avoid creases forming on the front side of the undergarment, and the need for sufficient absorption, may be found.

For example, the maximum front bond width may be in the range from 4 to 10 cm., such as from 6 to 10 cm.

Further, the absorbent assembly has a front portion maximum width being the maximum transversal width of the absorbent assembly in the front intermediate region of the undergarment. The front portion maximum width will hence be no less than the maximum front bond width. For example, the front portion maximum width may be greater than the maximum front bond width.

Thus, the front portion maximum width may, similar to the maximum front bond width, be no more than 11 cm. As such, it has been found that by restricting the front portion maximum width, the tendency of the undergarment to form unwanted creases towards the front of the wearer may be further reduced.

Optionally, the front portion maximum width in the front intermediary region may be no more than 10 cm, such as for example no more than 9 cm.

The front portion maximum width in the front intermediary region may for example be no less than 4 cm, such as no less than 5 cm or no less than 6 cm. For example, the front portion maximum width in the front intermediary region may be in the range from 4 to 10 cm, such as from 6 to 10 cm.

Optionally, in the front intermediate region, the maximum front bond width over the set of bonding members is substantially equal to the front portion maximum width of the absorbent assembly.

Further, the absorbent assembly may have a transversal assembly crotch width at the longitudinal location of the transversal crotch axis. For avoiding the formation of undesired creases on the front side of the undergarment, the transversal assembly crotch width may be no greater than the maximum front bond width. For example, the transversal assembly crotch width may be no greater than the front portion maximum width of the absorbent assembly in the front intermediate region.

Optionally, the transversal assembly crotch width of the absorbent assembly may be less than the maximum front bond width over the set of bonding members in the front intermediate region. As such, the absorbent assembly may be shaped to be further adapted to the body of the wearer, for increased comfort and fit of the undergarment.

Alternatively or in addition, the transversal assembly crotch width of the absorbent assembly may less than the front portion maximum width of the absorbent assembly in the front intermediate region.

This allows for forming the absorbent assembly such that the transversal width of the absorbent assembly increases as seen from the transversal crotch axis and in a longitudinal direction towards the front of the undergarment until reaching the maximum front bond width and/or the front portion maximum width. This may provide for even better fit to the user in combination with advantageous absorption properties.

The intermediate region of the undergarment has likewise a transversal undergarment crotch width at the longitudinal location of the transversal crotch axis. Optionally, the transversal undergarment crotch width may be substantially equal to the transversal assembly crotch width, i.e. the absorbent assembly extends transversally all the way between the leg openings of the undergarment at the longitudinal location of the transversal crotch axis.

In some variants, the transversal undergarment crotch width of the undergarment may be a minimum crotch width of the intermediate region. As such, the transversal crotch axis is located at the minimum crotch width of the intermediate region of the undergarment.

This allows for forming the absorbent assembly such that the transversal width of the absorbent assembly increases as seen from the transversal crotch axis and in a longitudinal direction towards the front of the undergarment until reaching the maximum front bond width and/or the front portion maximum width, and such that the transversal width of the absorbent assembly also increases as seen from the transversal crotch axis and in a longitudinal direction towards the rear of the undergarment until reaching a rear portion maximum width. This may provide for a good fit to the user in combination with advantageous absorption properties and be suitable for many models of undergarments.

For example, the transversal assembly crotch width may be in the range from 3 to 8 cm.

A first front portion length of the absorbent assembly may be defined along the longitudinal direction from the transversal crotch axis to the longitudinal position of the maximum front bond width. As such, the first front portion length may have an impact on the capacity of the undergarment to absorb a relatively high flow, and on the body fit and appearance of the undergarment when worn.

The absorbent assembly as a whole may extend longitudinally beyond the first front portion length. Thus, a total longitudinal length of the absorbent assembly in the front intermediate region could be equal to or greater than the first front portion length.

Optionally, the first front portion length may be less than 15 cm. Optionally, the first front portion length may be greater than 4 cm. For example, the first front portion length may be in the range from 4 to 15 cm, such that in the range from 4 to 12 cm. Thus, the undergarment as proposed herein may be designed to provide an absorbent assembly suitable for absorbing a moderate to heavy flow of body fluids, while not being unnecessary large.

In some variants, the absorbent assembly may extend longitudinally beyond the front intermediate region in a direction towards the front of the undergarment, i.e. the absorbent assembly may be partly arranged in the front region of the undergarment. For some such variants, a maximum front bond width over the set of bonding members in the front region of the undergarment may be no more than the maximum front bond width of the set of bonding members in the front intermediate region. Alternatively or in addition, for some such variants, a maximum width of the absorbent assembly in the front region of the undergarment is no more than the maximum width of the absorbent assembly in the front intermediate region.

In other variants, the absorbent assembly may extend no further than the front intermediate region in a direction longitudinally towards the front of the undergarment. That is, the absorbent assembly does not extend into the front region of the undergarment. This may be preferred so as to provide an undergarment where the position and extension of the absorbent assembly is configured for satisfactory performance for everyday use, and to be most useful for absorbing bodily fluids.

It will be understood that the absorbent assembly may extend not only in the front intermediate region of the undergarment, but also in the rear intermediate region of the undergarment, so as to be effective to absorb a flow of body fluids. However, unwanted folds or creases in the one or more fabric panels is less of a problem towards the rear side of the undergarment than towards the front side of the undergarment, due to the body shape. As such, more options may be available for forming the portion of the absorbent assembly positioned in the rear intermediate region than in the front intermediate region.

Optionally, the absorbent assembly extends longitudinally over at least part of the front intermediate region and part of the rear intermediate region.

A rear portion maximum width being the maximum transversal width of the absorbent assembly in the rear intermediate region may be no less than the front portion maximum width. A maximum rear bond width being the maximum transversal extension over the set of bonding members in the rear intermediate region may be no less than the maximum front bond width.

For example, a rear portion maximum width of the absorbent assembly in the rear intermediate region may be greater than the front portion maximum width of the absorbent assembly in the front intermediate region. A maximum rear bond width in the rear intermediate region may be greater than the maximum front bond width in the front intermediate region.

Optionally, the absorbent assembly may extend longitudinally towards the rear of the undergarment no further than the rear intermediate region. That is, the absorbent assembly does not extend into the rear region of the undergarment. As such, the absorbent assembly may be confined to the parts of the undergarment where it will be most useful for absorbing bodily fluids.

The total longitudinal length of the absorbent assembly may be configured depending on e.g. the model of the undergarment and taking the need for sufficient absorption into account.

Optionally, the maximum front bond width is at least 70%, such as for example at least 80% or at least 90% of the transversal width of the entire undergarment at the location of the maximum front bond width. As such, the one or more fabric panels forming the intermediate region of the undergarment may extend a relatively short distance beyond the sides of the absorbent assembly at the location of the maximum front bond width.

In some variants, the maximum front bond width of the set of bonding members may be substantially equal to the transversal width of the intermediate region at the location of the maximum front bond width. This implies that the set of bonding members are arranged over a width essentially corresponding to the width of the intermediate front region at the longitudinal location of the maximum front bond width.

Similarly, the maximum front width of the absorbent assembly may be at least 70%, such as for example at least 80% or at least 90% of the transversal width of the entire undergarment at the location of the maximum front width. In some variants, the maximum front width of the absorbent assembly may be substantially equal to the transversal width of the intermediate region of the undergarment at the location of the maximum front width.

As such, at least a greater portion of the width available for the absorbent assembly in the intermediate front region of the undergarment may be occupied by the absorbent assembly. For example, essentially the entire width available for the absorbent assembly in the intermediate front region may be occupied by the absorbent assembly.

The absorbent assembly may comprise a front edge directed towards the front of the undergarment, a rear edge directed towards the rear of the undergarment, and a pair of side edges, wherein each side edge is at least partly directed towards a respective leg opening and connects the front edge and the rear edge.

Each side edge may be at least partly arranged along the respective leg opening of the undergarment. With "along" is herein meant that the side edge follows the shape of the leg opening and is arranged adjacent or at the respective leg opening.

For example, each side edge may be at least partly arranged at the respective leg opening of the undergarment. This implies that the outer edge of the undergarment towards each leg opening at least partly comprises the side edge of the absorbent assembly.

For example, a minimum transversal assembly crotch width of the absorbent assembly, i.e. a minimum distance between the side edges of the absorbent assembly, may be equal to a minimum transversal undergarment crotch width of the intermediate region of the undergarment.

For example, each side edge of the absorbent assembly may be arranged at the respective leg opening of the undergarment in at least the front intermediate region.

For example, essentially the entire each side edge of the absorbent assembly may be arranged at the respective leg opening of the undergarment.

In other variants, each side edge may be at least partly arranged along the respective leg opening of the undergarment, and with a distance to the respective leg opening. For example, each side edge may in its entirety be arranged along a respecting leg opening, and with a distance to the leg opening.

Optionally, the set of bonding members is arranged to attach the absorbent assembly to the at least one out of the one or more of fabric panels at least along part of the side edges of the absorbent assembly. For example, the set of bonding members may be arranged to attach the absorbent assembly to the at least one out of the one or more of fabric panels along essentially the entire side edges of the absorbent assembly.

Optionally, the set of bonding members is arranged to attach the absorbent assembly to the at least one out of the one or more of fabric panels at least along part of the front edge of the absorbent assembly. For example, the set of bonding members may be arranged to fasten the absorbent assembly to the at least one out of the one or more of fabric panels along essentially the entire front edge of the absorbent assembly.

Optionally, the set of bonding members is arranged to attach the absorbent assembly to the at least one out of the one or more of fabric panels at least along part of the rear edge of the absorbent assembly. For example, the set of bonding members may be arranged to fasten the absorbent assembly to the at least one out of the one or more of fabric panels along essentially the entire rear edge of the absorbent assembly.

Thus, for example, in some variants the set of bonding members may be arranged to attach the absorbent assembly to the at least one out of the one or more fabric panels along the side edges, the front edge, and the rear edge of the absorbent assembly.

The shape of the absorbent assembly may be adapted for example to the design of the undergarment, and in view of the need for sufficient absorption and satisfactory fit.

In some variants, the side edges may comprise concave portions, as seen towards the central longitudinal axis y. For example, the side edges may be generally concave, thus allowing for the shape of the absorbent assembly being relatively wider towards the front and rear of the undergarment, and narrower at a crotch portion of the undergarment comprising the transversal crotch axis, as mentioned in the above.

In some variants, the front edge may be convex from the transversal crotch axis. As such, at the location of the central longitudinal axis y, the front edge may protrude towards the front of the undergarment beyond the side edges of the absorbent assembly. This may be beneficial for the fit of the undergarment to the body and contribute to the avoidance of unwanted creases in the fabric panels.

The rear edge of the absorbent assembly may be shaped so as to allow for suitable fit and comfort to a user.

As mentioned in the above, the absorbent assembly is permanently attached to one or more of the fabric panels by a set of bonding members.

For example, the set of bonding members may comprise one or more bonding lines. For example, one or more bonding lines may be arranged so as to extend along at least part of the side edges of the absorbent assembly, such as to extend along the side edges of the absorbent assembly in the intermediate region of the undergarment. Optionally, the one or more bonding line may be arranged so as to extend along essentially the entire side edges of the absorbent assembly.

Alternatively, or in addition, one or more bonding lines may be arranged so as to extend along at least part of the front edge of the absorbent assembly, such as so as to extend along essentially the entire front edge of the absorbent assembly.

Alternatively, or in addition, one or more bonding lines may be arranged so as to extend along at least part of the rear edge of the absorbent assembly, such as so as to extend along essentially the entire rear edge of the absorbent assembly.

The one or more bonding lines may be continuous bonding lines.

Optionally, the one or more bonding lines may be intermittent bonding lines.

The set of bonding members may comprise any suitable bonding member for accomplishing the permanent attachment of the absorbent assembly to the one or more fabric panels.

For example, the set of bonding members may comprise one or more adhesive bonding members, one or more mechanical bonding members, and/or a combination of adhesive and mechanical bonding members.

For example, the set of bonding members may comprise at least one adhesive bonding member. An adhesive bonding member may for example comprise an adhesive in the form of an adhesive joint. In another example, an adhesive bonding member may comprise an adhesive tape.

For example, the set of bonding members may comprise at least one mechanical bonding member. A mechanical bonding member may for example comprise a stitch or a seam.

The intermediate region of the undergarment may comprise at least one out of the one or more fabric panels, the fabric panel having an exterior side facing away from the wearer, and an interior side facing in a direction towards the wearer. The absorbent assembly may be located on the interior side of the at least one fabric panel.

As such, when the set of bonding member comprises a mechanical bonding member, the mechanical bonding member may be arranged so as to extend through the absorbent assembly and the at least one fabric panel. As such, the mechanical bonding member may be visible from the exterior side of the fabric panel.

As outlined in the above, the absorbent assembly comprises a wearer facing top layer, a moisture barrier, and at least two intermediate layers being superimposed along a height axis, perpendicular to the central longitudinal axis and the transversal crotch axis, between the top layer and the moisture barrier. As such, the absorbent assembly comprises at least three layers plus the moisture barrier, which in some variants is formed by a moisture barrier layer. Such an absorbent assembly may be particularly suited for a moderate to heavy flow of body fluids.

Optionally, the absorbent assembly comprises a wearer facing top layer, a moisture barrier, and at least three intermediate layers. As such, the absorbent assembly comprises at least four layers plus the moisture barrier, which in some variants is formed by a moisture barrier layer. Accordingly, the capacity of the absorbent assembly to absorb relatively high flows of body fluid may be further improved, for example to provide an absorbent assembly suited for a heavy flow of body fluids.

For example, the absorbent assembly may comprise no more than nine layers.

As such, the absorbent assembly may comprise two, three, four, five, six, seven or eight intermediate layers.

The longitudinal and transversal extension (i.e. the outer shape) of the absorbent assembly per se is defined by the mutual longitudinal and transversal extension of the wearer facing top layer and the moisture barrier.

In some variants, at least one of the at least two intermediate layers may have a longitudinal and transversal extension essentially coinciding with the extension of the absorbent assembly.

In some variants, at least two of the at least two intermediate layers may have a longitudinal and transversal extension essentially coinciding with the extension of the absorbent assembly.

For example, all of the intermediate layers of the absorbent assembly may have a longitudinal and transversal extension essentially coinciding with the extension of the absorbent assembly. I.e. all of the layers of the absorbent assembly have the same longitudinal and transversal extension.

In other variants, the longitudinal and/or transversal extension of at least one out of the three intermediate layers may differ from the longitudinal and/or transversal extension of the absorbent assembly.

Optionally, at the location of the maximum front bond width, at least three layers, such as at least four layers, of the absorbent assembly are all superimposed over one another over at least 70% of the maximum front bond width. For example, at least three layers, such as at least four layers, of the absorbent assembly may be superimposed over one another over at least 80%, or at least 90% of the maximum front bond width. Optionally, all of the layers of the absorbent assembly may all be superimposed over one another over at least 70% of the maximum front bond width, such as over at least 80% or at least 90 % of the maximum front bond width.

For example, the top layer may be a weft knit. In another example, the top layer is of a Jacquard technique. The top layer may for example have a basis weight of 80-200 gsm. Optionally, the top layer may be of polyester, elastane or blends thereof.

Optionally, one or more of the at least two intermediate layers is an absorbent layer. For example, when the absorbent assembly comprises at least three intermediate layers, at least two of the at least three intermediate layers may be absorbent layers.

The basis weight of the absorbent layer(s) may for example be in the range 200-350 gsm. For example, the absorbent layer(s) may be of polyester, polyamide, or a mixture thereof. Optionally, the absorbent layer is of a hydrophilic French terry material.

Optionally, one or more of the at least two intermediate layers may be a wicking layer. For example, the wicking layer may be arranged immediately adjacent the top layer.

Optionally, the basis weight of the wicking layer is 180-250 gsm. For example, the wicking layer may be of polyester, polyamid, elastane or a mixture thereof. For example, the wicking layer may be a jersey knit.

### BRIEF DESCRIPTION OF THE DRAWINGS

The disclosure will be described in greater detail below with reference to the figures shown in the appended drawings, wherein
- Fig. 1: shows a front view of a variant of an absorbent undergarment comprising an absorbent assembly;
- Fig. 2: shows a top view of the undergarment of Fig. 1 when in a flat laid-out state with the joints between the front rear region and the front region being removed; and
- Fig. 3: is a schematic cross-sectional view of a portion of the absorbent undergarment of Fig. 1 through the absorbent assembly.

### DETAILED DESCRIPTION

Different aspects of the present disclosure will be described more fully hereinafter with reference to the enclosed drawings. The embodiments of the absorbent undergarment disclosed herein can, however be realized in many different forms, such as different sizes and absorption levels, and should not be construed as being limited to the aspects set forth herein. In all figures in the following detailed description, the same reference numerals will be used to indicate the same elements.

As used herein, the term "absorbent undergarment" refers to garments that are worn and intended to be placed against the skin of the wearer to absorb and contain body fluids such as urine and vaginal fluids including menstrual fluid. The undergarment may for example be an underwear, underpants, a panty, or a swimwear of a fabric. The undergarment is intended for adult users. The undergarment may be intended for female use. The undergarment according to the present disclosure is washable, i.e. intended to be laundered or otherwise restored after use for reuse as a sanitary article.

By "washable" it is meant that the absorbent undergarment may be cleaned by laundering. The absorbent undergarment may thus be subjected to an aqueous solution containing detergent without losing its structural features. This aqueous solution may be heated as part of the laundering process, e.g. to 40°C or 60°C.

The term "fabric" as used in the present disclosure may refer to single or multiple layers of fabrics. The fabric may be knitted or woven.

By "permanently attached", it is meant that the absorbent assembly is not intended to be separated from the body fabric before, during, or after use. The absorbent assembly is not necessarily directly bonded to the fabric panels, but instead may be attached to the fabric via auxiliary parts such as leg opening edgings or reinforcement patches.

Knitting is a method of constructing a fabric by interlocking a series of loops of one or more yarns. There are two major classes of knitting namely warp and weft knitting.

Weaving is a method or process of interlacing two yarns of similar materials so that they cross each other at right angles to produce woven fabric. The warp yarns run lengthwise in the fabric and the filling threads (weft) or picks, run from side to side.

Jacquard is a system of weaving or knitting that utilizes a versatile pattern mechanism to produce intricate designs by using punch cards controlling the patterns produced. Jacquard knit may be valid for circular, flatbed, warp or weft knit.

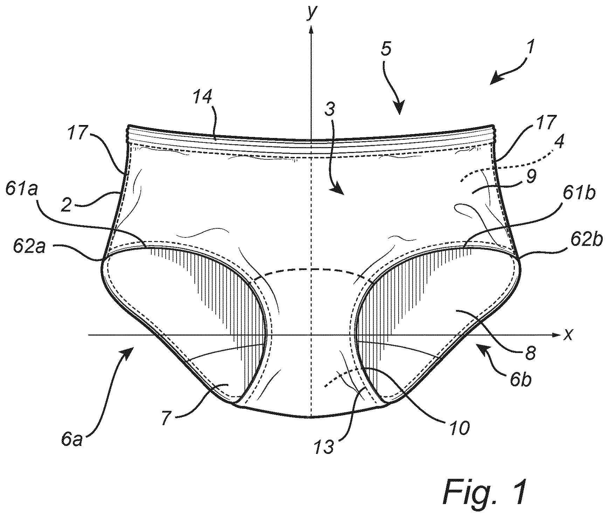

Fig. 1 illustrates as an example of a variant of a washable and reusable absorbent undergarment 1 in the form of a panty. As outlined in the above, the undergarment 1 as proposed herein may however have various designs, such as briefs, boxer, hipster, high waist, string, Brazilian, or other suitable undergarment designs. The undergarment 1 is designed primarily for female use.

The undergarment 1 may comprise one or more fabric panels 2 forming a front region 3, a back region 4 and an intermediate region 7 extending between the front and back regions 3, 4. The front region 3 and the back region 4 are joined such that the undergarment 1 forms a waist opening 5 and a pair of leg openings 6a, 6b. As such, the front region 3 will be visible from the front of the user when the undergarment 1 is worn, and the back region 4 will be visible from the back of the user when the undergarment 1 is worn.

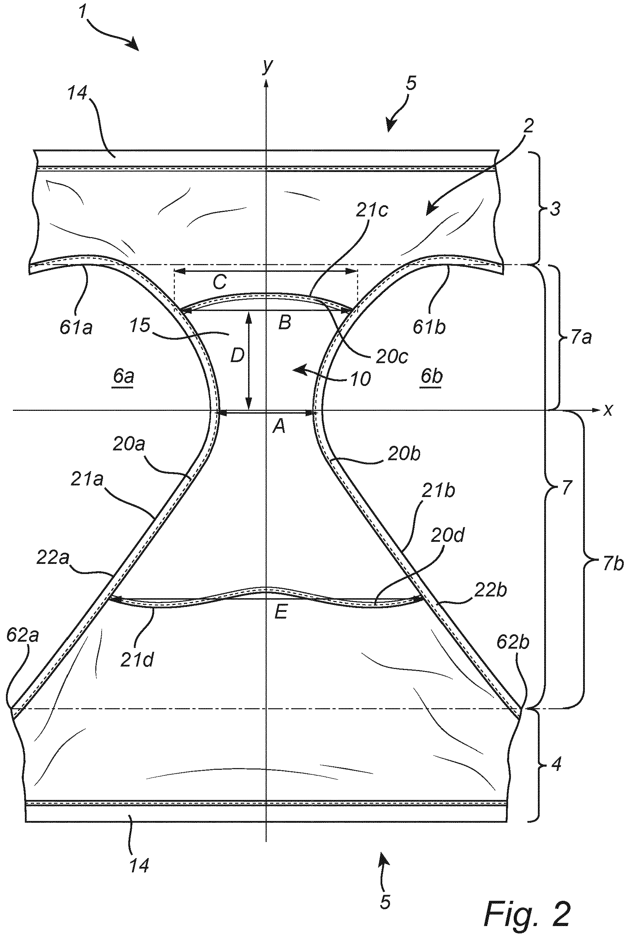

The undergarment of the Fig. 1 and Fig. 2 example comprises one fabric panel 2, which is cut to form the front region 3, the back region 4 and the intermediate region 7. However, in other variants, a plurality of fabric panels may be joined so as to form the front region 3, the back region 4 and the intermediate region 7. For example, the undergarment 1 may comprise a front panel, a back panel and an intermediate panel. The one or more fabric panels may all comprise the same fabric, giving the undergarment a unitary appearance, or the one or more fabric may comprise different fabrics, for example to provide aesthetically pleasing undergarments comprising e.g. lace fabric.

To join the front region 3 and the back region 4 so as to form the waist opening 5 and the leg openings 6a, 6b, a pair of side joints 17 may be provided, as in the illustrated example. The side joints 17 and any other joints joining the one or more fabric panels 2 may be conventional joints in the art, such as seams made by conventional adhesive and/or mechanical bonds such as conventional adhesive or stitching techniques.

A central longitudinal axis y of the undergarment 1 is defined along the one or more fabric panels 2 of the undergarment 1 from the back region 4 and towards the front region 3. As such, when an undergarment 1 is seen from the front side as in the example of Fig. 1, the central longitudinal axis y would be directed towards the waist opening 5 of the undergarment 1. However, when the undergarment 1 is seen from the back side (not shown), the central longitudinal axis y would be directed away from the waist opening 5 of the undergarment 1.

In Fig. 2, the absorbent undergarment 1 of Fig. 1 is shown in a flat laid-out state, where the side joints 17 of the undergarment 1 are removed. As seen in this laid-out state, the central longitudinal axis y is defined along the one or more fabric panels 2 and in a direction from the rear of the undergarment 1 towards the front of the undergarment 1. In Fig. 2, the absorbent undergarment 1 is shown from a wearer-facing side of the article 1. As illustrated in Fig. 1, the one or more fabric panels 2 will have an exterior side 9 facing away from the wearer, and an interior side 8 facing in a direction towards the wearer. Fig. 2 thus displays the undergarment 1 in a flat laid-out state as seen from the interior side 8.

Further, a transversal crotch axis x of the undergarment 1 is defined in a direction extending between the leg openings 6a, 6b and so as to divide the intermediate region 7 into a front intermediate region 7a extending longitudinally between the transversal crotch axis x and a front end 61a, 61b of each leg opening 6a, 6b, and a rear intermediate region 7b extending longitudinally between the transversal crotch axis x and a rear end 62a, 62b of each leg opening 6a, 6b, the transversal crotch axis x being perpendicular to the central longitudinal axis y.

As such, the transversal crotch axis x divides each leg opening 6a, 6b into a front portion of the leg opening 6a, 6b, which is intended to extend from the crotch of the user towards the front of the undergarment when the undergarment 1 is worn, and a rear portion of leg opening 6a, 6b, which is intended to extend from the crotch of the user towards the rear of the undergarment when the undergarment 1 is worn. As such, the transversal crotch axis x is to extend over a central crotch area of the user when the undergarment 1 is worn.

The absorbent undergarment 1 may comprise a waist band 14, arranged along the waist opening 5 of the absorbent undergarment 1.

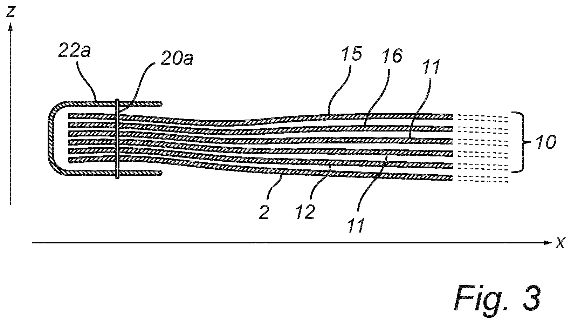

The undergarment 1 further comprises an absorbent assembly 10. As shown in Fig. 3, the absorbent assembly 10 comprises a wearer facing top layer 15, a moisture barrier 12, and at least two intermediate layers 11, 16 being superimposed between the top layer 15 and the moisture barrier 12 along a height axis z perpendicular to the central longitudinal axis y and the transversal crotch axis x. The layers 11, 12, 15, 16 of the absorbent assembly 10 will be further described in the below in relation to Fig. 3.

As seen in Fig. 2, in the illustrated undergarment 1, the absorbent assembly 10 is located on an interior side 8 of the at least one fabric panel 2. The absorbent assembly 10 is arranged over the transversal crotch axis x and so as to extend in the front intermediate region 7a, and in the rear intermediate region 7b.

Although in some variants the absorbent assembly 10 may extend longitudinally beyond the front intermediate region 7a in a direction towards the front of the undergarment 1, in the illustrated variant, the absorbent assembly 10 extends no further than the front intermediate region 7a in the longitudinal direction towards the front region 3 of the undergarment 1.

The longitudinal extension of the absorbent assembly 10 into the rear intermediate region 7b may depend for example on the design of the undergarment 1. In some variants, such as in the illustrated variant, the absorbent assembly 10 may extend no further than the rear intermediate region 7b in the longitudinal direction towards the rear region 4 of the undergarment 1.

As such, the absorbent assembly 10 may be confined to the intermediate region 7, wherein the need for absorbance is immediately prevalent. However, for example for undergarments intended primarily for night-time use, the absorbent assembly 10 may extend beyond the rear intermediate region 7b and into the rear region 4 of the undergarment 1, and/or beyond the front intermediate region 7a and into the front region 3 of the undergarment 1.

As exemplified by the illustrated undergarment 1, the absorbent assembly 10 forms a front edge 21c directed towards the front of the undergarment 1, a rear edge 21d directed towards the rear of the undergarment, and a pair of side edges 21a, 21b. Each side edge 21a, 21b is at least partly directed towards a respective leg opening 6a, 6b. Each side edge 21a, 21b, thus connects the front edge 21c and the rear edge 21d.

Each side edge 21a, 21b may be at least partly arranged along the respective leg opening 6a, 6b of the undergarment 1, such as at the leg opening 6a, 6b of the undergarment 1. In some variants, and in the illustrated example, the entire side edges 21a, 21b are arranged at the respective leg openings 6a, 6b of the undergarment 1, i.e. the side edges 21a, 21b follow the contour of the leg openings 6a, 6b of the undergarment 1.

The shape of the absorbent assembly 10 may be adapted to various absorption needs and to various designs of the undergarment 1, so as to provide sufficient absorption and satisfactory fit.

For example, the side edges 21a, 21b may comprise concave portions, as seen towards the central longitudinal axis y. For example, as in the illustrated variant, the entire side edges 21a, 21b are concave towards the central longitudinal axis y, i.e. curving inwards towards the central longitudinal axis y. Thus, the absorbent assembly 10 may be adapted to fit between the wearer's legs.

The front edge 21c of the absorbent assembly may similarly be shaped in view of the need for comfort and fit of the undergarment 1. In some variants, such as in the illustrated undergarment 1, the front edge may be convex from the transversal crotch axis x, i.e. curving away from the transversal crotch axis x. As illustrated in Fig. 2, at the location of the central longitudinal axis y, the front edge 21c may thus protrude towards the front of the undergarment 1 beyond the extension of the side edges 21a, b of the absorbent assembly 10. This may be beneficial for the fit of the undergarment to the body and contribute to the avoidance of unwanted creases in the fabric panels.

As mentioned in the above, each side edge 21a, 21b may at least partly be arranged at the respective leg opening 6a, 6b of the undergarment 1. This implies that the outer edge of the undergarment 1 forming each leg opening 6a, 6b at least partly comprises the side edge 21a, 21b of the absorbent assembly 10. For example, as in the illustrated variant, the outer edge of the undergarment 1 forming each leg opening 6a, 6b comprises the entire side edge 21a, 21b of the absorbent assembly 10. However, in other variants, the undergarment 1 may extend transversally beyond the absorbent assembly 10 in the directions towards each leg opening 6a, 6b in at least a part of the undergarment 1. For example, the side edges 21a, 21b of the absorbent assembly 10 may follow the leg openings 6a, 6b in a portion of the undergarment 1 adjacent the transversal crotch axis x, but the side edges 21a, 21b of the absorbent assembly 10 may deviate from the leg openings 6a, 6b in an area further to the front of the undergarment 1. In other words, the one or more fabric panels 2 may extend beyond the side edges 21a, 21b in directions away from the central longitudinal axis y. In such variants, the portions of the fabric panels 2 extending beyond the side edges 21a, 21b in directions away from the central longitudinal axis y may for example form leg portions of the undergarment 1. As such, the leg portions extending beyond the absorbent assembly 10 may adapt to and fold towards the wearer's legs, while the absorbent assembly 10 remains unfolded between the wearer's legs.

Further, when each side edge 21a, 21b is at least partly arranged at the respective leg opening 6a, 6b of the undergarment 1, as in the illustrated undergarment 1, a minimum assembly crotch width A of the absorbent assembly, i.e. a minimum distance between the side edges 21a, 21b of the absorbent assembly 10, may be equal to a minimum undergarment crotch width of the intermediate region 7 of the undergarment 1.

In some undergarment designs suitable for the absorbent undergarment proposed herein, the intermediate region 7 displays a relatively narrower crotch portion, which crotch portion comprises the transversal crotch axis x, and relatively wider front and rear portions towards the front and rear, respectively, of the crotch portion. As such, the absorbent assembly 10 may have a minimum transversal width being the transversal assembly crotch width A at the transversal crotch axis x, and the width of the absorbent assembly 10 may increase along the longitudinal axis towards the front of the undergarment, from the minimum transversal assembly crotch width A to a front portion maximum width C. Also, the width of the absorbent assembly 10 may increase along the longitudinal axis y towards the rear of the undergarment 1, from the transversal assembly crotch width A to a rear portion maximum width.

The absorbent assembly 10 is permanently attached to at least one out of the one or more fabric panels 2 in at least part of the intermediate region 7 by a set of one or more bonding members 20a, 20b, 20c, 20d.

The bonding members 20a, 20b, 20c, 20d may have any configuration suitable for permanently attaching the absorbent assembly to the one or more fabric panels 2.

Further, the bonding members 20a, 20b, 20c, 20d may be any type of bonding member suitable for accomplishing the permanent attachment of the absorbent assembly 10 to the one or more fabric panels 2. As such, the set of bonding members 20a, 20b, 20c, 20d may comprise one or more adhesive bonding members, such as adhesive layers, strings or dots, and/or adhesive tape, or one or more mechanical bonding members, such as rivets or stitches, and/or a combination of adhesive and mechanical bonding members. However, adhesive bonding members and/or conventional mechanical bonding members such as conventional seams and stiches may be preferred.

The set of bonding members 20a, 20b, 20c, 20d could be applied for example as a bonding pattern or layer formed over the surface of the absorbent assembly 10 facing the one or more fabric panels 2. As such, the set of bonding members may comprise an adhesive layer or a pattern of adhesive dots or mechanical stiches spread over the area of the surface of the absorbent assembly 10 facing the one or more fabric panels 2.

In another example, and as in the illustrated variant, the set of bonding members 20a, 20b, 20c, 20d may for example be arranged to permanently attach the absorbent assembly 10 to the at least one out of the one or more of the fabric panels 2 along at least part of one or more of the side edges 21a, 21b, the front edge 21c and the rear edge 21d.

For example, as in the illustrated variant, the set of bonding members 20a, 20b, 20c, 20d may be arranged to attach the side edges 21a, 21b, the front edge 21c and the rear edge 21d to the one or more fabric panels 2.

For example, and as in the illustrated undergarment 1, the set of bonding members may comprise one or more bonding lines 20a, 20b, 20c, 20d. As may be seen in the example of Fig. 2, one bonding line 20a, 20b may be arranged so as to extend along essentially each of the entire side edges 21a, 21b of the absorbent assembly 10. Further, in the illustrated example, one bonding line 20c is arranged to extend along essentially the entire front edge 21c and one bonding line 20d is arranged to extend along essentially the entire rear edge 20d.

Although in the illustrated variant, the bonding lines 20a, 20b, 20c, 20d are arranged to extend adjacent the respective edges 21a, 21b, 21c, 21d of the absorbent assembly 10, other variants may be envisaged where the bonding lines 20a, 20b, 20c, 20d are arranged at the respective edges 21a, 21b, 21c, 21d, e.g. so as to extend over the respective edges 21a, 21b, 21c, 21d.

The one or more bonding lines 20a, 20b, 20c, 20d may, as in the illustrated example, be continuous bonding lines, such as seams or continuous adhesive lines. In other variants, however, the one or more bonding lines may be intermittent bonding lines.

The bonding members 20a, 20b, 20c, 20d may be arranged to directly attach the absorbent assembly 10 to the one or more fabric panels 2. Alternatively, the bonding members 20a, 20b, 20c, 20d may be arranged to indirectly attach the absorbent assembly 10 to the one or more fabric panels 2. For example, the bonding members 20a, 20b, 20c, 20d may be arranged to attach the absorbent assembly 10 to the one or more fabric panels 2 via an auxiliary element such as for example an edging or a reinforcement patch.

Optionally, one or more of the bonding members 20a, 20b, 20c, 20d may be arranged so as to bond the layers 11, 12, 15, 16 (see Fig. 3) of the absorbent assembly 10 together while attaching the absorbent assembly 10 to the one or more fabric panels 2. Optionally, one or more of the bonding members 20a, 20b, 20c, 20d may be formed as part of e.g. a seam extending along an entire leg opening 6a, 6b of the intermediate portion.

In the illustrated variant, purely as an example, the intermediate portion 7 is provided with a side edging 22a, 22b along each leg opening 6a, 6b, and the bonding members 20a, 20b provided along the side edges 21a, 21b of the absorbent assembly 10 are formed by parts of the seams connecting the side edgings 22a, 22b to the one or more fabric panels 2 of the intermediate portion 7.

As proposed herein, in the front intermediate region 7a of the undergarment 1, a maximum front bond width B over the set of bonding members 20a, 20b, 20c, 20d is no more than 11 cm.

Thus, the maximum front bond width B is the maximum transversal width as measured over the set of bonding members 20a, 20b, 20c, 20d in the front intermediate region 7a. For example, and as in the illustrated variant, the maximum front bond width B may extend over the maximum transversal distance between a bonding line 20a along the first side edge 21a and a bonding line 20b along the second side edge 21b.

By restricting the maximum front bond width B as proposed herein, it is ensured that the one or more body panels 2 on the frontside of the undergarment 1 may adapt to the wearer's body without being unnecessarily hindered by the absorbent assembly 10, which, by virtue of the number of layers, is naturally less flexible than the one or more body panels 2. The maximum front bond width B is thus the maximum width along which the body panel or panels 2 in the front of the undergarment 1 will be restricted by the capacity of the absorbent assembly 10 to adapt to the wearer's body.

The maximum front bond width B may for example be no more than 10 cm, such as no more than 9 cm.

Still, the maximum front bond width B may be selected to be sufficiently large so as to achieve sufficient absorption capacity. For example, the maximum front bond width B may be no less than 4 cm, such as no less than 5 cm.

For example, and as in the illustrated variant, the maximum front bond width B may be in the range from 4 to 11 cm, such as from 4 to 10 cm, or from 5 to 9 cm.

Further, and as illustrated by the example of Fig. 2, the absorbent assembly 10 may have a front portion maximum width C being the maximum transversal width of the absorbent assembly 10 in the front intermediate region 7a.

As intimated in the above, the set of bonding members 20a, 20b, 20c, 20d need not extend all the way to the side edges 21a, 21b of the absorbent assembly 10. Hence, the front portion maximum width C may be greater than the maximum front bond width B over the set of bonding members 20a, 20b, 20c, 20d. This implies that, at the location of the maximum front bond width B, the absorbent assembly 10 may extend beyond the set of bonding members 20a, 20b, 20c, 20d in the transversal directions away from the central longitudinal axis y. However, in this case the outmost transversal portions of the absorbent assembly 10 thus not being bonded to the one or more fabric panel 2 is not believed to severely restrict the capacity of the one or more fabric panels 2 to adapt to the wearer's body.

As such, the front portion maximum width C of the absorbent assembly 10 may be greater than the maximum front bond width B. For example, the front portion maximum width C may be no more than 12 cm, as compared to the maximum front bond width B being no more than 11 cm.

In some variants, as exemplified in the drawings, the difference between the maximum front bond width B and the front portion maximum width C may essentially result from a seam allowance provided peripheral of the set of bonding members 20a, 20b, 20c, 20d. For example, in some variants the front portion maximum width C and the maximum front bond width B may differ by less than 1 cm, such as less than 0.5 cm or less than 0.3 cm.

However, in other variants, the maximum front bond width B may be substantially equal to the front portion maximum width C of the absorbent assembly. For example, when the maximum front bond width B extends over a bonding line 20a at the first side edge 21a and a bonding line 20b at the second side edge 21b, the maximum front bond width B and the front portion maximum width C may coincide.

In some variants, the front portion maximum width C is no more than 11 cm, such as no more than 10 cm, such as for example no more than 9 cm. The front portion maximum width C in the front intermediary region 7a may be no less than 4 cm, such as no less than 5 cm. For example, the front portion maximum width C in the front intermediary region 7a may be in the range from 4 to 10 cm.

Further, the absorbent assembly 10 has a transversal assembly crotch width A being the transversal width of the absorbent assembly 1 at the longitudinal location of the transversal crotch axis x.

The transversal assembly crotch width A may be no greater than the maximum front bond width B and/or the front portion maximum width C.

For example, the absorbent assembly 10 may have a substantially constant transversal width along the front intermediate portion 7a.

However, to improve the fit of the undergarment 1 to the body, the transversal assembly crotch width A may be less than the front portion maximum width C of the absorbent assembly 10. Further, the transversal assembly crotch width A may be less than the maximum front bond with B of the absorbent assembly 10.

As intimated in the above, the shape of the intermediate region 7 of the undergarment 1 may in some variants deviate from the shape of the absorbent assembly 10 along the leg openings 6a, 6b, i.e. the one or more body fabric panels 2 may extend beyond the side edges 21a, 21b of the absorbent assembly 10 towards the leg openings 6a, 6b. As such, a transversal undergarment crotch width of the undergarment 1 at the longitudinal location of the transversal crotch axis xmay differ from, e.g. be greater than, the transversal assembly crotch width A of the absorbent assembly 10.

In other variants, and as in the illustrated example, the transversal undergarment crotch width of the undergarment 1 at the longitudinal location of the transversal crotch axis x may be substantially equal to the transversal assembly crotch width A.

In variants where the intermediate portion 7 comprises a relatively narrower crotch portion and relatively wider front and rear portions, as for example in the illustrated variant, the transversal undergarment crotch width of the undergarment 1 may be a minimum transversal width of the intermediate region 7 of the undergarment 1.

For example, the transversal assembly crotch width A may be the range from 3 to 8 cm.

Further, a first front portion length D of the absorbent assembly 10 may be defined along the longitudinal direction y from the transversal crotch axis x to the longitudinal position of the maximum front bond width B over the set of bonding members 20a, 20b, 20c, 20d in the front intermediate region 7a. As such, the first front portion length D indicates the distance between the transversal crotch axis x and the maximum front bond width B.

Optionally, the first front portion length D may be less than 10 cm, such that in the range from 4 to 10 cm. As such, the shape and location of the absorbent assembly 10 in the undergarment 1 may be adapted so as to be useful for absorbing a moderate to heavy flow of body fluid without requiring an unnecessary large absorbent assembly 10. In the rear intermediate region 7b, a maximum rear bond width E being the maximum transversal width as measured over the set of bond members 20a, 20b, 20c, 20d in the rear intermediate region 7b may be defined. Due to anatomy, the formation of unwanted creases in the undergarment 1 is however not as prevalent on the rear side of the undergarment 1 as on the front side of the undergarment 1. Accordingly, the rear portion maximum width E may be more freely chosen so as to provide the desired absorption capacity and still provide satisfactory body fit towards the rear side of the wearer's body. Similarly, a maximum transversal rear width being the maximum transversal width of the absorbent assembly 10 in the rear intermediate region 7b may be relatively freely chosen.

As such, the maximum rear bond width E may be no less than the maximum front bond width B, and may for many variants be greater than the maximum front bond width B.

For example, and as in the illustrated variant, the absorbent assembly 10 may extend longitudinally towards the rear of the undergarment 1 no further than the rear intermediate region 7b. That is, the absorbent assembly 10 does not extend into the rear region 4 of the undergarment 1. As such, in these variants, the absorbent assembly 10 may be confined to the parts of the undergarment where it will be most useful for absorbing bodily fluids.

The total longitudinal length of the absorbent assembly 10 may be configured depending on e.g. the model of the undergarment and taking the need for sufficient absorption into account.

In some variants, when the set of bonding members comprises a mechanical bonding member, the mechanical bonding member may be arranged so as to extend through the absorbent assembly 10 and the at least one fabric panel 2. As such, the mechanical bonding member may be visible from the exterior side of the fabric panel 2.

Fig. 3 shows as an example of a schematic cross-sectional view of a portion of the intermediate region 7 of the absorbent undergarment 1 of Figs 1 and 2. The absorbent assembly 10 comprises a wearer facing top layer 15, a moisture barrier 12, and at least two intermediate layers 11, 16 being superimposed between the top layer 15 and the moisture barrier 12 along a height axis z perpendicular to the central longitudinal axis y and the transversal crotch axis x. In Fig. 3, the layers 11, 12, 15, 16 are illustrated as being spaced from for each other for better visibility. However, in practice the layers will be superimposed over one another, each layer contacting the neighboring upper layer and/or lower layer as seen in the height direction z.

In the variant of Fig. 3, three intermediate layers 11, 16, in this case two absorbent layers 11 and a wicking layer 16, are shown as an example. Further, the absorbent assembly 10 comprises a liquid-impermeable moisture barrier 12. The liquid-impermeable barrier 12 prevents liquid from leaking through the one or more intermediate layers 11 into the fabric panel 2. The liquid-impermeable barrier 12 may be in the form of a liquid-impermeable barrier layer 12 as illustrated in Fig. 3. In other variants however, the liquid-impermeable barrier 12 may be a coating on the wearer facing side of a fabric panel 2 or on a garment facing side of an absorbent layer 11. The absorbent assembly 10 further comprises a top layer 15 facing the skin of the user.

Further, in Fig. 3, an edging 22a and a bonding member 20a formed for example by a seam are illustrated as an example where the bonding member 20a joins the layers 11, 12, 15, 16 of the absorbent assembly 10 together and also attaches the absorbent assembly 10 to the fabric panel 2.

In other variants, the layers of the absorbent assembly 10 may be joined separately from (e.g. prior to) the attachment of the absorbent assembly 10 to the fabric panel 2.

The top layer 15 may be constructed of any suitable fabric, including naturally derived fibers selected from the group consisting of cotton, wool, silk, cellulose, regenerated cellulose, rayon, viscose, modal, lyocell, tencel, bamboo, hemp, flax, ramie, coir or banana. Alternatively, the top layer may be constructed of synthetic fibers selected from the group consisting of polyamide, acrylic, polyester or elastane, such as a mixture of polyester and elastane. Further, the top layer 15 may be constructed of a blend or a mixture of naturally derived and/or synthetic fibers. The materials used for construction of the top layer should be soft and non-irritating to the skin and be readily penetrated by any body fluids. According to the present disclosure, the top layer should be washable. The top layer may comprise between 20% and 100% naturally derived fibers, more preferably between 40% and 100% naturally derived fibers and most preferably between 60% and 100% naturally derived fibers. The top skin-facing layer 15 comprises a water-permeable material thus allowing the body fluids to migrate to the underlying absorbent layer or layers 11. The top layer 15 may have a basis weight of 80-200 gsm.

As exemplified in the variant illustrated in Fig. 3, a wicking layer 16 may be provided underneath the top layer 15. The wicking layer 16 has wicking features to allow the moisture to spread away from the wearer and into the absorbent layer or layers 11. Further, wicking enables an efficient spread of the moisture therefore allowing the moisture to be received in a wider area of the underlying one or more absorbent layers 11. This feature is particularly relevant for users suffering from stress incontinence as spurts of urine may cause the absorbent layer 11 to be saturated rapidly at the point of impact. By spreading the volume of the fluid over a wider receiving area in the absorbent layer 11, the wicking features of the wicking layer 16 increase the overall absorptive capacity of the absorbent layer. The wicking layer 16 may be constructed of any suitable fabric, including naturally derived fibers or mixtures thereof selected from the group consisting of cotton, wool, silk, cellulose, regenerated cellulose, rayon, viscose, modal, lyocell, tencel, bamboo, hemp, flax, ramie, coir or banana. Alternatively, the wicking layer 16 may be constructed of synthetic fibers or mixtures thereof selected from the group consisting of polyamide, acrylic, polyester or elastane. Further, the wicking layer 16 may be constructed of a blend or a mixture of naturally derived and/or synthetic fibers. According to the present disclosure, the wicking layer should be launderable. The wicking layer 16 may comprise between 20% and 100% naturally derived fibers, more preferably between 40% and 100% naturally derived fibers and most preferably between 60% and 100% naturally derived fibers. The basis weight of the wicking layer may be 180-250 gsm.

The absorbent assembly 10 of the absorbent undergarment 1 may contain one or multiple absorbent layers 11, capable of absorbing liquid and releasing the liquid during laundering. Release of the absorbed liquid is beneficial as it enables the absorbent undergarment 1 to be restored for reuse. Reusable absorbent undergarments provide a more sustainable alternative to the commonly used disposable hygiene articles that are not intended to be re-used.

The absorbent layer or layers 11 may comprise any material capable of absorbing fluid, such as woven or nonwoven microfiber or polymer knits, fabric formed from hydrophilic fibers, absorbent fibers or powders. The absorbent layer or layers 11 may be of natural or synthetic fibers as described above for the other ingoing layers but may be of polyester and polyamide and containing odor treatment. The odor treatment may be silver ions, copper ions and zeolites or Polyhexamethylene biguanide, PHMB.

The absorbent layer or layers 11 may also comprise a material having an open cell porous structures such as high loft or synthetic fibers having reservoir properties. The absorbent layer may comprise between 20% and 100% naturally derived fibers, more preferably between 40% and 100% naturally derived fibers and most preferably between 60% and 100% naturally derived fibers. The basis weight of each absorbent layer 11 may be 200-350 gsm. There may be one or more absorbent layers 11 in the absorbent assembly 10. For example, as in the illustrated variant, the absorbent assembly 10 may comprise two absorbent layers 11.

The liquid-impermeable barrier 12 may comprise any suitable material or combinations of material that prevents liquid from migrating from the absorbent assembly 10 to the fabric layer 2. The liquid-impermeable barrier 12 may, as in the illustrated variant, be a liquid-impermeable barrier layer and may comprise a hydrophobic woven or nonwoven material having inherent hydrophobic properties, or that has been treated to become hydrophobic.

Examples of hydrophobic materials for treating the barrier layer are polymers such as silicone, polyurethane and combinations thereof. The liquid-impermeable barrier layer 12 may comprise a microporous polymer film, e.g. a polyethylene, PTFE or polyurethane film, or combinations thereof. Laminates of polymer films and nonwoven materials may also be used. Alternatively, the liquid-impermeable barrier 12 may be a coating of a moisture-impermeable material. The coating may be a polymer such as urethane wax provided for example on the surface facing away from the wearer of an absorbent layer 11, or on the surface facing towards the wearer of a fabric panel 2. The liquid-impermeable barrier 12 may be of polyurethane.

The liquid-impermeable barrier 12 is preferably breathable, to allow vapor to escape from the absorbent undergarment 1, while preventing liquid from passing through the fabric layer. Further, the liquid-impermeable barrier 12 may be constructed of an elastic material, thus providing the absorbent assembly 1 with the required flexibility to adapt to the user's anatomy and movements. This improved fit increases the wearer's comfort during use and helps to prevent leakage from migrating through the undergarment's leg openings 6a, 6b.

The fabric of the one or more fabric panels 2 may be constructed of any suitable fabric, including naturally derived fibers and mixtures thereof selected from the group consisting of cotton, wool, silk, cellulose, regenerated cellulose, rayon, viscose, modal, lyocell, tencel, bamboo, hemp, flax, ramie, coir or banana. Alternatively, the fabric may be constructed of synthetic fibers or mixtures thereof selected from the group consisting of polyamide, acrylic, polyester. Further, the fabric may be constructed of a blend or a mixture of naturally derived and/or synthetic fibers. The fibers may be recycled fibers. The fabric may comprise a stretchable fabric, e.g. elastane, so that the absorbent undergarment can provide a firm fit while at the same time adapting to the wearer's movements thus preventing any leakage from migrating through the leg openings and keeping the absorbent assembly in place. The fabric is preferably breathable to allow vapor to escape from the wearer's skin and from the absorbent structure.

The disclosure may be varied within the scope of the appended claims. For example, the materials and dimensions used for the different layers forming the absorbent insert may be varied, as indicated above.

## Claims

1. A washable and reusable undergarment (1), comprising one or more fabric panels (2) forming a front region (3), a back region (4) and an intermediate region (7) extending between the front and back regions (3, 4), the front region (3) and the back region (4) being joined such that the undergarment (1) forms a waist opening (5) and a pair of leg openings (6a, 6b), wherein a central longitudinal axis (y) of the undergarment (1) is defined along the one or more fabric panels (2) of the undergarment (1) from the back region (4) and towards the front region (3), and a transversal crotch axis (x) of the undergarment (1) is defined in a direction extending between the leg openings (6a, 6b) and so as to divide the intermediate region (7) into a front intermediate region (7a) extending longitudinally between the transversal crotch axis (x) and a front end (61a, 61b) of each leg opening (6a, 6b), and a rear intermediate region (7b) extending longitudinally between the transversal crotch axis (x) and a rear end (62a, 62b) of each leg opening (6a, 6b), the transversal crotch axis (x) being perpendicular to the central longitudinal axis (y);
the undergarment (1) further comprising an absorbent assembly (10) comprising a wearer facing top layer (15), a moisture barrier (12), and at least two intermediate layers (11, 16) being superimposed along a height axis (z), perpendicular to the longitudinal axis (y) and the transversal crotch axis (x), between the top layer (15) and the moisture barrier (12),
and wherein
the absorbent assembly (10) is permanently attached to at least one out of the one or more of fabric panels (2) in at least part of the intermediate region (7) by a set of one or more bonding members (20a, 20b, 20c, 20d) wherein, in the front intermediate region (7a) of the undergarment (1), a maximum front bond width (B) as measured transversally over the set of bonding members (20a, 20b, 20c, 20d) attaching the absorbent assembly (10) to the one or more fabric panels (2) is no more than 11 cm.

2. The absorbent undergarment according to claim 1, wherein the maximum front bond width (B) over the set of bonding members (20a, 20b, 20c, 20d) in the front intermediate region (7a) is in the range from 4 to 10 cm.

3. The undergarment according to claim 1 or 2, wherein the absorbent assembly (10) has a front portion maximum width (C) being the maximum transversal width of the absorbent assembly (10) in the front intermediate region (7a), the front portion maximum width (C) being no more than 11 cm.

4. The undergarment according to claim 3, wherein the front portion maximum width (C) is in the range from 4 to 10 cm.

5. The undergarment according to any one of the preceding claims, wherein, in the front intermediate region (7a), the maximum front bond width (B) over the set of bonding members (20a, 20b, 20c, 20d) is substantially equal to the front portion maximum width (C) of the absorbent assembly (10).

6. The undergarment according to any one of the preceding claims, wherein the absorbent assembly (10) has a transversal assembly crotch width (A) at the longitudinal location of the transversal crotch axis (X), the transversal crotch with (A) being no greater than the maximum front bond width (B) over the set of bonding members (20a, 20b, 20c, 20d) in the front intermediate region (7a) and/or no greater than the front portion maximum width (C) of the absorbent assembly (10) in the front intermediate region (7a).

7. The absorbent undergarment according to any one of the preceding claims, wherein the transversal assembly crotch width (A) is in the range from 3 to 8 cm.

8. The absorbent undergarment according any one of the preceding claims, wherein a first front portion length (D) of the absorbent assembly (10) is defined along the longitudinal direction (y) from the transversal crotch axis (X) to the longitudinal position of the maximum front bond width (B) over the set of bonding members (20a, 20b, 20c, 20d) in the front intermediate region (7a), wherein the first front portion length (D) is in the range from 4 to 15 cm.

9. The absorbent undergarment according to any one of the preceding claims, wherein a maximum front bond width over the set of bonding members (20a, 20b, 20c, 20d) in the front region (3) of the undergarment (1) is no more than the maximum front bond width (B) of the set of bonding members (20a, 20b) in the front intermediate region (7a).

10. The absorbent undergarment according to any one of the preceding claims, wherein, in the front region (3) of the undergarment (1), a maximum width of the absorbent assembly (10) is no more than the maximum width (C) of the absorbent assembly (10) in the front intermediate region (7a).

11. The absorbent undergarment according to any one of the claims 1 to 8, wherein the absorbent assembly (10) extends no further than the front intermediate region (7a) in a direction longitudinally towards the front of the undergarment (1).

12. The absorbent undergarment according to any one of the preceding claims, wherein the maximum front bond width (B) of the set of bonding members (20a, 20b, 20c, 20d) is substantially equal to the transversal width of the intermediate region (7) at the location of the maximum front bond width (B).

13. The absorbent undergarment according to any one of the preceding claims, wherein the absorbent assembly (10) comprises a front edge (21c) directed towards the front of the undergarment (1), a rear edge (21d) directed towards the rear of the undergarment (1), and a pair of side edges (21a, 21b), each side edge (21a, 21b) being at least partly directed towards a respective leg opening (6a, 6b), and connecting the front edge (21c) and the rear edge (21d).

14. The absorbent undergarment according to claim 13, wherein each side edge (21a, 21b) is at least partly arranged at the respective leg opening (6a, 6b) of the undergarment (1)

15. The absorbent undergarment according to claim 13 or 14, wherein the set of bonding members (20a, 20b, 20c, 20d) is arranged to fasten the absorbent assembly (10) to the at least one out of the one or more of fabric panels (2) at least along part of the side edges (21a, 21b) of the absorbent assembly (10).

## Patentansprüche

1. Waschbare und wiederverwendbare Unterwäsche (1), umfassend eine oder mehrere Stoffbahnen (2), die einen vorderen Bereich (3), einen hinteren Bereich (4) und einen Zwischenbereich (7), der sich zwischen dem vorderen und hinteren Bereich (3, 4) erstreckt, bilden, wobei der vordere Bereich (3) und der hintere Bereich (4) so zusammengefügt sind, dass die Unterwäsche (1) eine Taillenöffnung (5) und ein Paar Beinöffnungen (6a, 6b) bildet, wobei eine mittlere Längsachse (y) der Unterwäsche (1) entlang der einen oder mehreren Stoffbahnen (2) der Unterwäsche (1) vom hinteren Bereich (4) und zum vorderen Bereich (3) definiert ist, und eine querverlaufende Schrittachse (x) der Unterwäsche (1) in einer Richtung definiert ist, die sich zwischen den Beinöffnungen (6a, 6b) und derart erstreckt, dass sie den Zwischenbereich (7) in einen vorderen Zwischenbereich (7a), der sich längslaufend zwischen der querverlaufenden Schrittachse (x) und einem vorderen Ende (61a, 61b) jeder Beinöffnung (6a, 6b) erstreckt,
und einen hinteren Zwischenbereich (7b) unterteilt, der sich längslaufend zwischen der querverlaufenden Schrittachse (x) und einem hinteren Ende (62a, 62b) jeder Beinöffnung (6a, 6b) erstreckt, wobei die querverlaufende Schrittachse (x) senkrecht zur mittleren Längsachse (y) ist; wobei die Unterwäsche (1) weiter eine absorbierende Anordnung (10) umfasst, die eine dem Träger zugewandte obere Schicht (15), eine Feuchtigkeitsbarriere (12) und mindestens zwei Zwischenschichten (11, 16) umfasst, die entlang einer Höhenachse (z), senkrecht zur Längsachse (y) und der querverlaufenden Schrittachse (x), zwischen der oberen Schicht (15) und der Feuchtigkeitsbarriere (12) übereinander gelagert sind,
und wobei
die absorbierende Anordnung (10) an mindestens einem Teil des Zwischenbereichs (7) durch einen Satz von einem oder mehreren Verbindungselementen (20a, 20b, 20c, 20d) dauerhaft mit mindestens einer der einen oder mehreren Stoffbahnen (2) verbunden ist, wobei im vorderen Zwischenbereich (7a) der Unterwäsche (1) eine maximale vordere Bandbreite (B), quer über den Satz von Verbindungselementen (20a, 20b, 20c, 20d) gemessen, die die absorbierende Anordnung (10) mit der einen oder den mehreren Stoffbahnen (2) verbinden, nicht mehr als 11 cm beträgt.

2. Absorbierende Unterwäsche nach Anspruch 1, wobei die maximale vordere Bandbreite (B) über den Satz von Verbindungselementen (20a, 20b, 20c, 20d) im vorderen Zwischenbereich (7a) im Bereich von 4 bis 10 cm liegt.

3. Unterwäsche nach Anspruch 1 oder 2, wobei die absorbierende Anordnung (10) eine maximale Breite des vorderen Abschnitts (C) aufweist, die die maximale querverlaufende Breite der absorbierenden Anordnung (10) im vorderen Zwischenbereich (7a) ist, wobei die maximale Breite des vorderen Abschnitts (C) nicht mehr als 11 cm beträgt.

4. Unterwäsche nach Anspruch 3, wobei die maximale Breite des vorderen Abschnitts (C) im Bereich von 4 bis 10 cm liegt.

5. Unterwäsche nach einem der vorstehenden Ansprüche, wobei die maximale vordere Bandbreite (B) im vorderen Zwischenbereich (7a) über den Satz von Verbindungselementen (20a, 20b, 20c, 20d) im Wesentlichen gleich der maximaler Breite des vorderen Abschnitts (C) der absorbierenden Anordnung (10) ist.

6. Unterwäsche nach einem der vorstehenden Ansprüche, wobei die absorbierende Anordnung (10) eine querverlaufende Anordnungsschrittbreite (A) an der längslaufenden Stelle der querverlaufenden Schrittachse (X) aufweist, wobei die querverlaufende Anordnungsschrittbreite (A) nicht größer ist als die maximale vordere Bandbreite (B) im vorderen Zwischenbereich (7a) über den Satz von Verbindungselementen (20a, 20b, 20c, 20d) und/oder nicht größer als die maximale Breite des vorderen Abschnitts (C) der absorbierenden Anordnung (10) im vorderen Zwischenbereich (7a).

7. Absorbierende Unterwäsche nach einem der vorstehenden Ansprüche, wobei die querverlaufende Anordnungsschrittbreite (A) im Bereich von 3 bis 8 cm liegt.

8. Absorbierende Unterwäsche nach einem der vorstehenden Ansprüche, wobei eine erste Vorderabschnittlänge (D) der absorbierenden Anordnung (10) entlang der Längsrichtung (y) von der querverlaufenden Schrittachse (X) zur Längsposition der maximalen vorderen Bandbreite (B) über den Satz von Verbindungselementen (20a, 20b, 20c, 20d) im vorderen Zwischenbereich (7a) definiert ist, wobei die erste Vorderabschnittlänge (D) im Bereich von 4 bis 15 cm liegt.

9. Absorbierende Unterwäsche nach einem der vorstehenden Ansprüche, wobei eine maximale vordere Bandbreite über dem Satz von Verbindungselementen (20a, 20b, 20c, 20d) im vorderen Bereich (3) der Unterwäsche (1) nicht größer ist als die maximale vordere Bandbreite(B) des Satzes von Verbindungselementen (20a, 20b) im vorderen Zwischenbereich (7a).

10. Absorbierende Unterwäsche nach einem der vorstehenden Ansprüche, wobei im vorderen Bereich (3) der Unterwäsche (1) eine maximale Breite der absorbierenden Anordnung (10) nicht größer ist als die maximale Breite (C) der absorbierenden Anordnung (10) im vorderen Zwischenbereich (7a).

11. Absorbierende Unterwäsche nach einem der Ansprüche 1 bis 8, wobei sich die absorbierende Anordnung (10) in einer Richtung längslaufend zur Vorderseite der Unterwäsche (1) nicht weiter als der vordere Zwischenbereich (7a) erstreckt.

12. Absorbierende Unterwäsche nach einem der vorstehenden Ansprüche, wobei die maximale vordere Bandbreite (B) des Satzes von Verbindungselementen (20a, 20b, 20c, 20d) an der Stelle der maximaler vorderen Bandbreite (B) im Wesentlichen gleich der querverlaufenden Breite des Zwischenbereichs (7) ist.

13. Absorbierende Unterwäsche nach einem der vorstehenden Ansprüche, wobei die absorbierende Anordnung (10) einen vorderen Rand (21c), der zur Vorderseite der Unterwäsche (1) ausgerichtet ist, einen hinteren Rand (21d), der zur Hinterseite der Unterwäsche (1) ausgerichtet ist, und ein Paar Seitenränder (21a, 21b) umfasst, wobei jeder Seitenrand (21a, 21b) zumindest teilweise zu einer jeweiligen Beinöffnung (6a, 6b) ausgerichtet ist und den vorderen Rand (21c) und den hinteren Rand (21d) verbindet.

14. Absorbierende Unterwäsche nach Anspruch 13, wobei jeder Seitenrand (21a, 21b) zumindest teilweise an der jeweiligen Beinöffnung (6a, 6b) der Unterwäsche (1) angeordnet ist.

15. Absorbierende Unterwäsche nach Anspruch 13 oder 14, wobei der Satz von Verbindungselementen (20a, 20b, 20c, 20d) eingerichtet ist, um die absorbierende Anordnung (10) an der mindestens einen der einen oder mehreren Stoffbahnen (2) zumindest entlang eines Teils der Seitenränder (21a, 21b) der absorbierenden Anordnung (10) zu befestigen.

## Revendications

1. Un sous-vêtement lavable et réutilisable (1), comprenant un ou plusieurs panneaux de tissu (2) formant une région avant (3), une région arrière (4) et une région intermédiaire (7) s'étendant entre les régions avant et arrière (3, 4), la région avant (3) et la région arrière (4), étant assemblées de sorte que le sous-vêtement (1) forme une ouverture à la taille (5) et une paire d'ouvertures aux jambes (6a, 6b), où un axe longitudinal central (y) du sous-vêtement (1) est défini le long du un ou plusieurs panneaux de tissu (2) du sous-vêtement (1) depuis la région arrière (4) et vers la région avant (3), et un axe transversal d'entrejambe (x) du sous-vêtement (1) est défini dans une direction s'étendant entre les ouvertures aux jambes (6a, 6b) et de façon à diviser la région intermédiaire (7) en une région intermédiaire avant (7a) s'étendant longitudinalement entre l'axe transversal d'entrejambe (x) et une extrémité avant (61a, 61b) de chaque ouverture aux jambes (6a, 6b), et une région intermédiaire arrière (7b) s'étendant longitudinalement entre l'axe transversal d'entrejambe (x) et une extrémité arrière (62a, 62b) de chaque ouverture aux jambes (6a, 6b), l'axe transversal d'entrejambe (x) étant perpendiculaire à l'axe longitudinal central (y) ;
le sous-vêtement (1) comprenant en outre un ensemble absorbant (10) comprenant une couche supérieure (15) faisant face au porteur, un obstacle à l'humidité (12), et au moins deux couches intermédiaires (11, 16) superposées le long d'un axe de hauteur (z), perpendiculaire à l'axe longitudinal (y) et à l'axe transversal d'entrejambe (x), entre la couche supérieure (15) et l'obstacle à l'humidité (12),
et dans lequel
l'ensemble absorbant (10) est fixé de façon permanente à au moins l'un du un ou plusieurs panneaux de tissu (2) dans au moins une partie de la région intermédiaire (7) par un ensemble d'un ou plusieurs éléments de liaison (20a, 20b, 20c, 20d) où, dans la région intermédiaire avant (7a) du sous-vêtement (1), une largeur de liaison avant maximale (B) telle que mesurée transversalement sur l'ensemble d'éléments de liaison (20a, 20b, 20c, 20d) fixant l'ensemble absorbant (10) à l'un ou plusieurs panneaux de tissu (2) ne dépasse pas 11 cm.

2. Le sous-vêtement absorbant selon la revendication 1, dans lequel la largeur de liaison avant maximale (B) sur l'ensemble d'éléments de liaison (20a, 20b, 20c, 20d) dans la région intermédiaire avant (7a) est comprise entre 4 et 10 cm.

3. Le sous-vêtement selon la revendication 1 ou 2, dans lequel l'ensemble absorbant (10) a une largeur maximale de partie avant (C) étant la largeur transversale maximale de l'ensemble absorbant (10) dans la région intermédiaire avant (7a), la largeur maximale de partie avant (C) ne dépassant pas 11 cm.

4. Le sous-vêtement selon la revendication 3, dans lequel la largeur maximale de partie avant (C) est comprise entre 4 et 10 cm.

5. Le sous-vêtement selon l'une des revendications précédentes, dans lequel, dans la région intermédiaire avant (7a), la largeur de liaison avant maximale (B) sur l'ensemble d'éléments de liaison (20a, 20b, 20c, 20d) est sensiblement égale à la largeur maximale de partie avant (C) de l'ensemble absorbant (10).

6. Le sous-vêtement selon l'une des revendications précédentes, dans lequel l'ensemble absorbant (10) a une largeur d'ensemble d'entrejambe transversale (A) à la position longitudinale de l'axe transversal d'entrejambe (X), la largeur d'ensemble d'entrejambe transversale (A) n'étant pas supérieure à la largeur de liaison avant maximale (B) sur l'ensemble d'éléments de liaison (20a, 20b, 20c, 20d) dans la région intermédiaire avant (7a) et/ou pas supérieure à la largeur maximale de partie avant (C) de l'ensemble absorbant (10) dans la région intermédiaire avant (7a).

7. Le sous-vêtement absorbant selon l'une des revendications précédentes, dans lequel la largeur d'ensemble d'entrejambe transversale (A) est comprise entre 3 et 8 cm.

8. Le sous-vêtement absorbant selon l'une des revendications précédentes, dans lequel une première longueur de partie avant (D) de l'ensemble absorbant (10) est définie le long de la direction longitudinale (y) depuis l'axe transversal d'entrejambe (X) jusqu'à la position longitudinale de la largeur de liaison avant maximale (B) sur l'ensemble d'éléments de liaison (20a, 20b, 20c, 20d) dans la région intermédiaire avant (7a), où la première longueur de partie avant (D) est comprise entre 4 et 15 cm.

9. Le sous-vêtement absorbant selon l'une des revendications précédentes, dans lequel une largeur de liaison avant maximale sur l'ensemble d'éléments de liaison (20a, 20b, 20c, 20d) dans la région avant (3) du sous-vêtement (1) n'est pas supérieure à la largeur de liaison avant maximale (B) de l'ensemble d'éléments de liaison (20a, 20b) dans la région intermédiaire avant (7a).

10. Le sous-vêtement absorbant selon l'une des revendications précédentes, dans lequel, dans la région avant (3) du sous-vêtement (1), une largeur maximale de l'ensemble absorbant (10) n'est pas supérieure à la largeur maximale (C) de l'ensemble absorbant (10) dans la région intermédiaire avant (7a).

11. Le sous-vêtement absorbant selon l'une des revendications 1 à 8, dans lequel l'ensemble absorbant (10) ne s'étend pas plus loin que la région intermédiaire avant (7a) dans une direction longitudinalement vers l'avant du sous-vêtement (1).

12. Le sous-vêtement absorbant selon l'une des revendications précédentes, dans lequel la largeur de liaison avant maximale (B) de l'ensemble d'éléments de liaison (20a, 20b, 20c, 20d) est substantiellement égale à la largeur transversale de la région intermédiaire (7) à l'emplacement de la largeur de liaison avant maximale (B).

13. Le sous-vêtement absorbant selon l'une des revendications précédentes, dans lequel l'ensemble absorbant (10) comprend un bord avant (21c) orienté vers l'avant du sous-vêtement (1), un bord arrière (21d) orienté vers l'arrière du sous-vêtement (1), et une paire de bords latéraux (21a, 21b), chaque bord latéral (21a, 21b) étant au moins partiellement orienté vers une ouverture aux jambes (6a, 6b) respective, et reliant le bord avant (21c) et le bord arrière (21d).

14. Le sous-vêtement absorbant selon la revendication 13, dans lequel chaque bord latéral (21a, 21b) est au moins partiellement disposé à l'ouverture aux jambes (6a, 6b) respective du sous-vêtement (1).

15. Le sous-vêtement absorbant selon la revendication 13 ou 14, dans lequel l'ensemble d'éléments de liaison (20a, 20b, 20c, 20d) est disposé pour fixer l'ensemble absorbant (10) à au moins un ou plusieurs panneaux de tissu (2) au moins le long d'une partie des bords latéraux (21a, 21b) de l'ensemble absorbant (10).
